# EUROPEAN PATENT APPLICATION

(11) **EP 2 113 257 A1**
(43) Date of publication of application: **04.11.2009**
(21) Application number: 08425304.6
(22) Date of filing: 30.04.2008
(51) Int. Cl.: A61K 49/00, G01N 33/00, G01N 33/48, G01N 33/50, G01N 33/52, G01N 33/574, A61P 35/00, A61K 31/785, A61K 31/722, A61K 38/02

(54) **Polyelectrolyte with positive net charge for use as medicament and diagnostic for cancer**

(71) Applicant: Consorzio per il Centro di Biomedica Moleculare Scrl, 34012 Basovizza (TS) (IT)
(72) Inventor: Krol, Silke c/o Consorzio per il Centro di, 34012 Basovizza (Trieste) (IT)
(74) Representative: Spadaro, Marco

(57) **Abstract**

The present invention relates to the medical field, in particular to the treatment and diagnosis of cancer. More in particular, the present invention relates to the use of polyelectrolytes with positive net charge, in particular cationic polyelectrolytes such as polyallamine, chitosan or poly-L-lysine as anticancer agents and as diagnostic agents for the detection and/or treatment of cancer cells in body tissues. These agents are useful as theranostic agents.

## Description

### Technical Field

The present invention relates to the medical field, in particular to the treatment and diagnosis of cancer.

More in particular, the present invention relates to the use of polyelectrolytes with positive net charge, in particular cationic polyelectrolytes as anticancer agents and as diagnostic agents for the detection and/or treatment of cancer cells in body tissues.

### Background Art

Leukemia is a broad term covering a spectrum of diseases, which are clinically and pathologically split into *acute* and *chronic*forms.

Acute leukemia is characterized by the rapid increase of immature blood cells. This crowding makes the bone marrow unable to produce healthy blood cells. Acute forms of leukemia can occur in children and young adults. Immediate treatment is required in acute leukemias due to the rapid progression and accumulation of the malignant cells, which then spill over into the bloodstream and spread to other organs of the body.

Chronic leukemia is distinguished by the excessive build up of relatively mature, but still abnormal, blood cells. Typically taking months to years to progress, the cells are produced at a much higher rate than normal cells, resulting in many abnormal white blood cells in the blood. Chronic leukemia mostly occurs in older people, but can theoretically occur in any age group. Whereas acute leukemia must be treated immediately, chronic forms are sometimes monitored for some time before treatment to ensure maximum effectiveness of therapy.

Furthermore, the diseases are classified into lymphoblastic or lymphocytic leukemias, which indicate that the cancerous change took place in a type of marrow cell that normally goes on to form lymphocytes, and myeloid or myelogenous leukemias, which indicate that the cancerous change took place in a type of marrow cell that normally goes on to form red blood cells, some types of white cells, and platelets.

Combining these two classifications provides a total of four main categories:

**Table 1**

| Four major kinds of leukemia | | |
|---|---|---|
| **Cell type** | **Acute** | **Chronic** |
| **Lymphocytic leukemia** (or "lymphoblastic") | Acute lymphocytic leukemia (ALL) | Chronic lymphocytic leukemia (CLL) |
| **Myelogenous leukemia** (also "myeloid" "nonlymphocytic") or | Acute myelogenous leukemia (AML) | Chronic myelogenous leukemia (CML) |

Types outside these main categories include hairy cell leukemia.

**Table 2**

| Comparison of leukemia types | | | |
|---|---|---|---|
| | | **5-year** | |
| **Type** | **Occurrence** | **survival rate** | **Overall treatment** |
| **Acute lymphocytic leukemia** | Most common type of leukemia in young children. This disease also affects adults, especially those age 65 and older. | 85% in children and 50% in adults | Bone marrow and systemic disease control, prevention of spreading, e.g. to CNS |
| **Chronic lymphocytic leukemia** | Most often affects adults over the age of 55. It sometimes occurs in younger adults, but it almost never affects children. 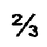 of affected are men. | 75% | Incurable |
| **Acute myelogenou s leukemia** | Occurs more commonly in adults than in children, and more commonly in men than women. | 40% | Bone marrow and systemic disease control, specific treatment of CNS, if involved |
| **Chronic myelogenou** | Occurs mainly in adults. A very small number of children | 90% | |
| **s leukemia** | also develop this disease. | | |
| **Hairy cell leukemia** | About 80% of affected people are adult men. No reported cases in young children. | 96% to Incurable, but 100% at easily treatable ten years | |

### Treatment options for leukemia

### Acute Lymphocytic Leukemia (ALL)

Proper management of ALL focuses on control of bone marrow and systemic (whole-body) disease as well as prevention of cancer at other sites, particularly the central nervous system (CNS).

Treatment for acute leukemia can include chemotherapy, steroids, radiation therapy, intensive combined treatments (including bone marrow or stem cell transplants), and growth factors.

### Chronic Lymphocytic Leukemia (CLL)

While generally considered incurable, CLL progresses slowly in most cases.

### Refractory CLL

"Refractory" CLL is a disease that no longer responds favourably to treatment. In this case more aggressive therapies are considered.

CLL is probably incurable by present treatments. But, fortunately, a large group of CLL patients do not require therapy.

Treatment of AML consists primarily of chemotherapy

### Chronic Myelogenous Leukemia (CML)

### Chronic phase CML is treated with inhibitors of tyrosine kinase,

### Blast crisis

Blast crisis carries all the symptoms and characteristics of either acute myelogenous leukemia or acute lymphoblastic leukemia, and has a very high mortality rate. This stage can most effectively be treated by a bone marrow transplant after high-dose chemotherapy.

### Hairy Cell Leukemia (HCL)

### Several treatments are available, and successful control of the disease is common.

Hairy cell leukemia is an incurable, indolent blood disorder in which mutated, partly matured B cells accumulate in the bone marrow.

The general medical literature, such as **PORTER, et al.** The Merck Manual. Merck, from which the background art is taken, provides an ample review of the treatment and diagnosis of leukemia and cancer in general.

Diagnosis of leukemia requires laboratory tests involving white blood cells count, however, bone marrow examination should often, if not always be performed. The latter requires biopsy or needle examination, which is mostly problematic to the patient, especially pediatric patients.

There is still the need of a diagnostic tool for a fast diagnosis of leukemia, possibly avoiding painful or complicated intervention on the patient.

Moreover, there is still the need for a fast, reliable diagnostic tool, which avoids sample preparation.

Cancer therapy is still a difficult task for the person expert in this field. Other than surgery, cancer therapy uses complex therapeutic protocols with anticancer drugs and combinations of said drugs and/or radiotherapy. Cancer therapy is mostly affected by severe side effects. Another strongly felt need is a medicament for treating cancer or a medicament for supplementing or enhancing or integrating cancer therapy, which is easy to manage and has reduced side effects.

In MARIKOVSKY, Y., et al. Distribution and Modulation of Surface Charge of Cells from Human Leukemia-Lymphoma lines at Various Stages of Differentiation. Cancer . 1986, vol.58, p.2218-2223, it is reported the use of cationized ferritin (CF) to label electrical charge surface density of hematopoietic cells at various stages of differentiation, showing a correlation between the CF density/distribution and the stage of lymphoid differentiation. Treatment with retinoic acid (RA) seems to prevent CF-induced formation of CF patches. Some correlation between the distribution of surface anionic sites and the malignant potential of human leukemic cells lines could be detected. The revelation of leukemic cells requires a laborious preparation of the sample.

CHANANA, M., et al. Interaction of Polyelectrolytes and their Composites with Living Cells. Nano Letters. 2005, vol.5, no.12, p.2605-12. The Authors study interaction and toxicity between polyelectrolytes and living cells. Toxicity was found to be influenced by all of factors such as contact area, charge, transplantation site and most important the cell type in contact with the polyelectrolyte and cannot be tested easily in a model. The goal of this work is to study cytotoxicity of polyelectrolytes in order to find suitable ones for use as coating in immunoprotected transplantation, so to say, to find polyelectrolytes with reduced or minimized or null toxicity. However, the Authors conclude that a general statement about cytotoxicity of a polyelectrolyte is not possible and that the use of a model system to investigate reactions to polymers remains mainly artificial.

SIGURDSON, C., et al. Prion Strain Discrimination Using Luminescent Conjugated Polymers. Nature Methods. 2007, vol.42, no.12, p.1023-1030. disclose the use of luminescent conjugated polymers for characterizing prion strains.

YEUNG, T., et al. Membrane Phosphatidylserine Regulates Surface Charge and Protein Localization. Science. 2008, vol.319, p.210-213. develop a biosensor to study the subcellular distribution of phosphatidylserine.

Several methods for diagnosing leukemia exist. For a review see The Merck Manual. 18th edition. Edited by PORTER, ROBERT S., et al. Merck & Co., 2008.

Some methods are based on the determination of gene expression (WO 2008/19872 A (RICHTER, GUNTHER) 21.02.2008 , WO 2006/89233 A (WYETH) 26.08.2006 , JP 2007244377 A (OKAYAMA UNIV) 27.09.2007 ) or molecular marker determination (US 2007287163 A (GEUIJEN ET AL.) 13.12.2007 , US 2007264261 A (NUVELO INC.) 15.11.2007.

All these methods are complex and require sample preparation.

The need of a fast, easy and reliable method for diagnosis of cancer, in particular leukemia, still exists.

The need of an easier method for treating cancer is also felt.

### Disclosure of Invention

It has been found that a polyelectrolyte bearing a positive net charge (in the following also said polycation) selectively enters cancer cells or cells in the process of malignant differentiation while it is excluded from healthy cells.

Advantageously, said polycation does not enter healthy cells even upon exposition of said cells for 24 h.

The selective uptake of the polyelectrolyte according to the present invention provides a fast and reliable diagnostic tool, which can advantageously be applied to body samples without complex sample preparation being necessary.

The polyelectrolyte according to the present invention is also capable of selectively killing the tumour cell.

Therefore, it is an object of the present invention a polyelectrolyte having a positive net charge for use as antitumor agent for cancer.

Another object of the present invention is a polyelectrolyte having a positive net charge for use as diagnostic agent for cancer.

The present invention provides the use of the above polyelectrolytes as theranostic agents.

These and other objects of the present invention will now be illustrated in detail also by means of examples and Figures.

In the Figures:

Figure 1: Polyelectrolyte (PAH) labelled with a fluorophore. Amount of stained cells (HL60, lymphocytes of leukemic and healthy person) after 15 min of incubation.

Figure 2: number of living HL60 cells against incubation time with or without FITC-PAH treatment.

### Modes for Carrying Out the Invention

According to the present invention, a polyelectrolyte having a positive net charge is also intended as a cationic polyelectrolyte or polycation.

The polyelectrolyte of the present invention, when used as a medicament, must be admitted for administration to a patient, being human or animal.

Generally, the polyelectrolytes according to the present invention bear ionisable groups such as primary amines, imines, quaternized amines (ammonium groups).

Examples of polyelectrolytes according to the present invention are: protamine, poly(disulfide amine), poly(amidoamine), poly(allylamine) and its derivatives, poly(glycoamidoamine), polyethylenimine (PEI), poly (lactic-co-glycolic acid)-poly ethyleneimine, linear polyethylenimine-β-poly(ethylene glycol)-β-polyethylenimine triblock copolymers, polyethylenimine-alt-poly(ethylene glycol) copolymers, hyperbranched polyether polyols partially functionalized with quaternary or tertiary ammonium groups, guanidinylated poly(propylene imine), dendrimers, 4-amino-1,8-naphthalimide, RGD-PEI, poly(2-dimethylamino ethylamino)phosphazene, PEG-folate coated poly(2-dimethylaminoethylamine-co-diaminobutane)phosphazene (poly(DMAEA-co-BA)phosphazene)-based polyplexes, cationic polyphosphazenes, cationic polymethacrylates, quaternized epsilon-caprolactone, cationized gelatine, poly[bis(2-chloroethyl) ether-*alt*-1,3-bis[3-(dimethylamino)propyl]urea] quaternized, poly-lysine (L, D (enantiomer) and L,D (racemic mixture)), dimethylmaleic acid-melittin-polylysine conjugate, poly-arginine (L, D (enantiomer) and L,D (racemic mixture)), poly-histidine and other poly-(amino acids) or poly-peptides, polyornithine, poly(diallyldimethylammonium chlorides) (poly-DADMAC) and their N-methyl-N-vinylacetamide derivates, α,β-poly(aspartylhydrazide) (PAHy), α,β-poly(aspartylhydrazide) bound to 3-(carboxypropyl)trimethylammonium chloride (CPTACI) (PAHy-CPTA), dextran-spermine polycation, chitosan and its derivatives, poly(2-ethyl-2-oxazoline) and its derivatives.

The polycations according to the present invention are commercially available via common suppliers or can be prepared in the laboratory according to common general knowledge of those of ordinary skill in the art.

In a preferred embodiment of the present invention, the polyelectrolyte is selected from the group consisting of poly-allylamine (PAH), chitosan and poly-L-lysine (PLL).

For the purpose of diagnostic use according to the present invention, the polyelectrolyte is labelled with a detectable label. According to a preferred embodiment of the invention, the detectable label will bind to the amine group of the polyelectrolyte. According to a more preferred embodiment, the detectable label will be a fluorophore or a chromophore.

In a general embodiment of the present invention, any sample from a subject suspected to suffer cancer disease or in need to confirm to suffer such a disease is suitable for the diagnosis according to the present invention. Blood is the preferred sample because it is the easiest to obtain and it stains cancer cells wherever found. For example, cancer cells are detected in blood samples of leukemia patients (bone marrow affected) but also in the blood of a patient with diagnosed melanoma (skin cancer) with the risk of metastasis. The method of diagnosis according to the present invention provides contacting the body sample with the polyelectrolyte herein disclosed and revealing the irreversible adhesion of said polyelectrolyte to the cancer cell or the internalization of said polyelectrolyte into said cancer cell.

In case of diagnostic application of the present invention, a preferred embodiment provides the detection of cancer cells in blood samples. In this case, cancer cells to be detected are non-adhesive cancer cells.

In an especially preferred embodiment, cancer cells to be detected are leukemia cells or invading cancer cells (detectable in blood because they leave the primary tumour and settle somewhere, causing metastasis). In this embodiment, the method of the invention is very convenient, since only a fast centrifugation is needed to separate the lymphocytes.

Other preferred embodiments relate to the detection of cancer cells from melanoma, liver and breast cancer.

A particular embodiment of the invention relates to the detection of metastatic cells. Detecting said cells in body circulation, for example blood or lymph circle is of critical importance for the management and cure of cancer disease.

In a still further embodiment, the present invention discloses a method for treating cancer comprising:
a. obtaining a blood sample from a subject affected by said cancer,
b. contacting said blood sample with a blood purification system whereby cancer cells contained in said blood sample are contacted with a solution of a polycation according to the present invention, whereby said polycation enters said cancer cells,
c. removing said cancer cells, to provide a purified blood sample,
d. returning said blood sample to said subject.

In an alternative embodiment, said method comprises:
a. obtaining a blood sample from a subject affected by said cancer,
b. contacting said blood sample with a blood purification system whereby cancer cells contained in said purification system are contacted with a solution of a polycation according to the present invention, whereby said polycation enters said cancer cells,
c. returning said blood sample to said subject.

Another embodiment of the present invention provides a method for treating an isolated blood sample from a subject affected by cancer comprising:
a. contacting said blood sample with a blood purification system whereby cancer cells contained in said blood sample are contacted with a solution of a polycation according to the present invention, whereby said polycation enters said cancer cells,
b. removing said cancer cells, to provide a purified blood sample.

Another embodiment of the present invention provides a method for treating an isolated blood sample from a subject affected by cancer comprising:
a. contacting said blood sample with a blood purification system whereby cancer cells contained in said purification system are contacted with a solution of a biodegradable polycation according to the present invention, whereby said polycation enters said cancer cells.

Depending on the kind of polycation used, the above methods for treating cancer or treating an isolated blood sample will be adopted.

If a non-biodegradable polycation is used according to the present invention, the cancer cells, after having incorporated the polycation, will be separated from blood. If a biodegradable polycation is used, blood containing cancer cells incorporating the polycation can be returned to the subject or the cells can be anyway separated from blood, before returning it to the subject.

The skilled person will be able to select the suitable polycation according to the common knowledge in the field, however, the following table indicates some examples.

| | |
|---|---|
| Potentially cytotoxic | Non-cytotoxic or low potential of cytotoxicity |
| polyethylenimine (PEI), | cationized gelatine |
| poly(diallyldimethylammonium chloride) (poly-DADMAC) | dextran-spermine polycation |
| polyallylamine | chitosan and its derivatives |
| poly(amidoamine) | poly-lysine (L, D (enantiomer) and L,D (racemic mixture)), |
| Poly-2-dimethylaminoethylamino (poly-DMAEA) | poly-Arginine (L, D (enantiomer) and L,D (racemic mixture)) |
| | poly-Histidine and other poly-(amino acids) or poly-peptides |
| | polyornithine, and their N-methyl-N-vinylacetamide derivates |
| | poly (lactic-co-glycolic acid) |
| | dendrimers |
| | hyperbranched polyether polyols partially functionalized with quaternary or tertiary ammonium groups |
| | cationic polymethacrylates |
| | quaternized epsilon-caprolactone |
| | dimethylmaleic acid-melittin-polylysine conjugate |
| | poly(disulfide amine) |
| | spermine |
| | poly(glycoamidoamine) |
| | α,β-poly(aspartylhydrazide) (PAHy) |
| | protamine |

Reduced cytotoxicity compared to the original polycation:linear polyethylenimine-β-poly(ethylene glycol)-β-polyethylenimine triblock copolymers, polyethylenimine-alt-poly(ethylene glycol) copolymers, guanidinylated poly(propylene imine), 4-Amino-1,8-naphthalimide, RGD-PEI, poly(2-dimethylamino ethylamino)phosphazene, PEG-folate coated poly(2-dimethylaminoethylamine-co-diaminobutane)-phosphazene, cationic polyphosphazenes, (poly(DMAEA-co-BA)phosphazene)-based polyplexes, poly[bis(2-chloroethyl) ether-alt-1,3-bis[3-(dimethylamino)propyl]urea] quaternized, α,β-poly(aspartylhydrazide) bound to 3-(carboxypropyl)trimethyl-ammonium chloride (CPTACI) (PAHy-CPTA, , poly(2-ethyl-2-oxazoline) and its derivatives.

However, cytotoxicity is not so general to state because it depends strongly on the test conditions and the cell types. Block-co-polymers of a potentially cytotoxic polymer can show a reduced toxicity. Therefore, a preliminary test on cytotoxicity is advisable.

Preferably, in the embodiment comprising the use of the cytotoxic polycation of the present invention, said polycation is labelled as disclosed before, for example with a fluorescent label. Separation of cancer cells incorporating the labelled polycation is made with any method known in the art, for example with a flow cytometer.

In the embodiment comprising the use of biodegradable polyelectrolyte, like chitosan or PLL, the blood sample so treated is returned to the subject where the cancer cells undergo apoptosis and will be removed by the body.

In the treatment providing the separation of cancer cells, it is convenient to provide the polycation with a detectable label as above explained, in order to facilitate separation of cancer cells after incorporation of the labelled polycation.

However, a detectable label can also be used if the cancer cells, incorporating the suitable polycation, are returned to the subject, in order to perform a follow up of the treatment through imaging or any other technique. In this case, a non-toxic label is used, such as carotene or other chromophores.

In another embodiment of the present invention, a suitable polycation can be directly administered to a subject, according to well-known techniques for chemotherapy. Guidance can be found in the general common knowledge, for example The Merck Manual of Diagnosis and Therapy, Remington's Pharmaceutical Sciences, last edition, Mack Publishing and Co.

### The following example further illustrates the invention.

### Example

### Material and Methods:

### Staining of the cancer cells

To determine the binding specificity of positively charged polyelectrolyte (poly-allylamine (PAH), chitosan, Poly-L-lysine (PLL), etc.) to the cancer cells, the human leukemia (HL60, acute promyelocytic leukemia) cell line as well as the lymphocytes, granulocytes and whole blood of leukemic (n=3) and healthy volunteers (n=3) were incubated with FITC-PAH (fluorescein isothiocyanate- polyallylamine; concentration: 2 mg/ml) for 2 min, 5 min, 15 min, 30 min, 1 h and 2 h incubated at 37°C and 5% CO₂.

### Analysis of the data

For the number of stained cells was determined in HL60 cells where all cells were supposed to be malign and for the lymphocytes fraction of a patient with diagnosed leukemia. The incubation was in both cases for 10 min under standard cell culture conditions (37°C, 5% CO₂). For the lymphocyte incubation a sample preparation was necessary prior to the treatment. From the whole blood sample, collected from the patient and stabilized by citrate, by Ficoll centrifugation the fraction containing the lymphocytes was separated from the monocytes and other blood components. The lymphocyte fraction was immediately after separation cultured in medium. The cell counting was always performed by two independent persons. In case of HL60 cells the experiments were repeated for 12 times and for each time at least 2 images were analyzed. For the lymphocyte analysis experiment were repeated for 4 times and for each time 4 or more images were analyzed.

### Survival rate of HL60 cells

In order to determine the survival of human leukemia cells after polyelectrolyte treatment the HL60 were incubated for 15 min, 1 h or 2.5 h in presence of the polyelectrolyte under otherwise standard culture conditions (37°C, 5% CO₂). It was found that significant amount of the cells adhere to the plate surface and there was a separate counting for the dead/live assay with PI (propidium iodide, stains dead cells red) staining with adherent and suspension cells.

### Results:

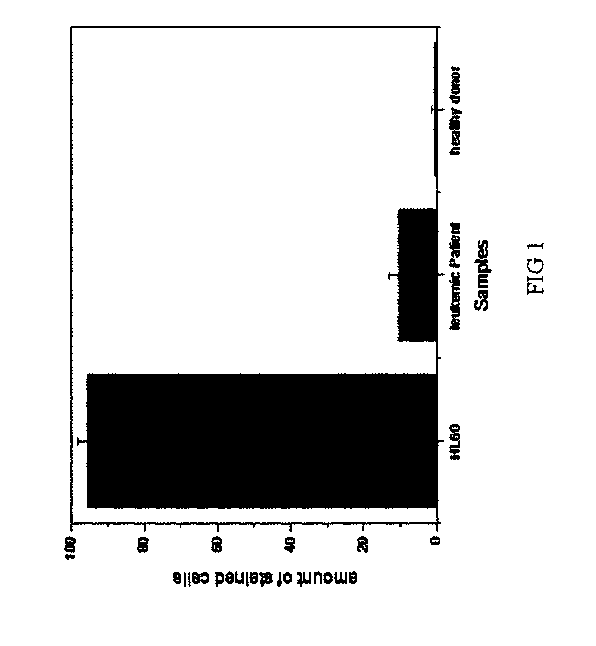

The polycations used according to the present invention are able to enter easily in non-adhesive cancer cells, like in case of leukemia or invading cancer cells, while they do not affect normal blood cells (figure 1). The increased permeability inside the cells is also observable for other cancer cells like melanoma, liver, and breast cancer.

It was found that FITC-PAH is binding to the plasma membrane and the cytoskeletons, but in some cases also to the nucleus and the DNA. Mostly binding the strong negativity of heterochromatin nucleoli region of the nucleus within 2 min incubation. After 5 min the polyelectrolytes are seen mostly in the cytoplasm and the nucleus. After 15 min incubation almost all the nucleus are stained with FITC-PAH. A live-dead assay with Propidium lodide (PI), a dye intercalating selectively in the DNA of dead or apoptotic cells, showed that most of the cells incubated FITC-PAH are dead. No competitive effect of PI and the polycation is expected because both are positively charged but the binding site to the DNA is different. PI is intercalating between the bases while the polymer is electrostatically bound to the negatively charged phosphate group of the DNA. After 1 h incubation, we see that all the HL60 cancer cells are in apoptosis, indicated by protruding vesicular structures. After 2 h of incubation with polyelectrolyte all cells are destructed and only fragments are present. Addition of FITC-PAH under microscope, showed that the polyelectrolyte enters the cells very quickly. Within 30 s polyelectrolytes are crossing the plasma membrane and staining the cytoplasm allowing the visualization of internal cell structure.

When FITC-PAH are add under microscope on whole blood, granulocytes and lymphocytes from leukemic patients and healthy volunteers, lymphocytes and monocytes (granulocytic component) of leukemic patient show similar phenomenon as HL60 cell line as most of them take up the polyelectrolyte within 30 s and binds mostly to the plasma membrane and cytoskeleton. After 5 min, 1 h and 2 h incubation we see the same effect as the HL60 cell line that all the cytoplasm and nucleus is totally stained. In patients only the leukemic lymphocytes were stained, but not the healthy ones. Both, lymphocytes and granulocytes of the healthy donor are impermeable to FITC-PAH. Not even overnight incubation with FITC-PAH make the cells permeable which indicates as well that the polyelectrolyte is not cytotoxic for the blood cells but it is highly cytotoxic for the endothelial cells (blood vessels) A systemic therapy with PAH is excluded because of the risk to damage irreversible the blood vessel or the myocardium but an ex vivo treatment in a blood purification system with strict control that no polycation enters the body after treatment will have a high therapeutic potential. Alternatively we can use polycations like chitosan which are potentially not cytotoxic and in this case a systemic application is possible.

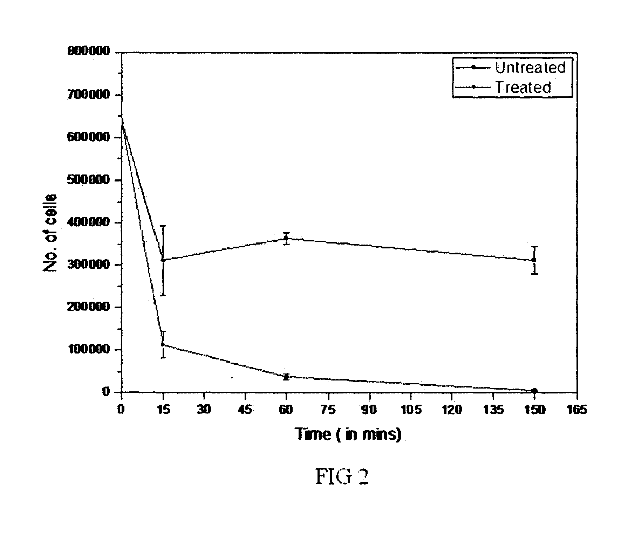

The staining of the cancer cells is very fast and extremely bright as can be seen by fluorescence micrograph. The amount of cells which are stained by FITC-PAH after 10 min were compared. In the case of HL60 cells around 95% have uptake the polycation while in case of the lymphocytes of a patient with diagnosed leukemia 12% of the cells are affected. In a lymphocyte sample of a healthy donor the amount of stained cells is 0.5 %. Moreover it was found that the polyelectrolyte which is entering exclusively the cancer cells are leading to their death (approx. 90 %, fig. 2). So the suggested method can serve as a theranostic system, identifying the diseased cells and at the same time selectively killing them.

Interesting in this context if the cells which are affected by the FITC-PAH survive on long-terms or if the incubation with the polyelectrolyte could offer also a therapeutic application. In several incubation experiments the survival rate of the incubated cells were tested. The survival rate of cells after 2.5 h in presence of FITC-PAH is negligible (fig.2).

### Potential application

The detection of leukemia cells can be in a fast and disposable test kit with high reliability. In a microchamber the dry fluorescent or even chromophore labelled polycation is immobilized in a gel. In contact with a blood sample the polyelectrolyte is solving in the serum and entering in the leukemic cells. Imaging under a microscope will give after 1 min the number of cells in which the polyelectrolyte will be able to enter. The cost is very low because the polycation (PAH) used for the test kit is very cheap.

### Industrial Application

The present invention relates to the medical field, in particular to the diagnosis and cure of cancer.

## Claims

1. A polyelectrolyte having a positive net charge for use as antitumor agent for cancer.

2. The polyelectrolyte according to claim 1, which is selected from the group consisting of protamine, poly(disulfide amine), poly(amidoamine), poly(allylamine) and its derivatives, poly(glycoamidoamine), polyethylenimine (PEI), poly (lactic-co-glycolic acid)-poly ethyleneimine, linear polyethylenimine-β-poly(ethylene glycol)-β-polyethylenimine triblock copolymers, polyethylenimine-alt-poly(ethylene glycol) copolymers, hyperbranched polyether polyols partially functionalized with quaternary or tertiary ammonium groups, guanidinylated poly(propylene imine), dendrimers, 4-Amino-1,8-naphthalimide, RGD-PEI, poly(2-dimethylamino ethylamino)phosphazene, PEG-folate coated poly(2-dimethylaminoethylamine-co-diaminobutane)phosphazene (poly(DMAEA-co-BA)phosphazene)-based polyplexes, cationic polyphosphazenes, cationic polymethacrylates, quaternized epsilon-caprolactone, cationized gelatine, poly[bis(2-chloroethyl) ether-*alt*-1,3-bis[3-(dimethylamino)propyl]urea] quaternized, poly-lysine (L, D (enantiomer) and L,D (racemic mixture)), dimethylmaleic acid-melittin-polylysine conjugate, poly-Arginine (L, D (enantiomer) and L,D (racemic mixture)), poly-Histidine and other poly-(amino acids) or poly-peptides, polyornithine, poly(diallyldimethylammonium chlorides) (poly-DADMAC) and their N-methyl-N-vinylacetamide derivates, α,β-poly(aspartylhydrazide) (PAHy), α,β-poly(aspartylhydrazide) bound to 3-(carboxypropyl)trimethylammonium chloride (CPTACI) (PAHy-CPTA), dextran-spermine polycation, chitosan and its derivatives, poly(2-ethyl-2-oxazoline) and its derivatives.

3. The polyelectrolyte according to any one of claims 1-2, which is labelled with a detectable label.

4. The polyelectrolyte according to any one of claims 1-3, wherein said cancer comprises non-adhesive cancer cells.

5. The polyelectrolyte according to any one of claims 1-3, wherein said cancer is selected from the group consisting of leukaemia, melanoma, invading cancer cells, liver and breast cancer.

6. A cationic polyelectrolyte for use as diagnostic agent for revealing cancer cells in a (isolated) tissue of a subject.

7. The polyelectrolyte according to claim 6, which is selected from the group consisting of polyallylamine, chitosan, poly-L-lysine.

8. The polyelectrolyte according to any one of claims 6-7, wherein said cancer comprises non-adhesive cancer cells.

9. The polyelectrolyte according to claim 8, wherein said cancer is selected from the group consisting of leukaemia, melanoma, invading cancer cells, liver and breast cancer.

10. A method for treating an isolated blood sample from a subject affected by cancer comprising:
a. contacting said blood sample with a blood purification system whereby cancer cells contained in said blood sample are contacted with a solution of a cytotoxic polycation of any one of claims 1-2, whereby said polycation enters said cancer cells,
b. removing said cancer cells, to provide a purified blood sample.

11. A method for treating an isolated blood sample from a subject affected by cancer comprising:
a. contacting said blood sample with a blood purification system whereby cancer cells contained in said purification system are contacted with a solution of a non-cytotoxic biodegradable polycation according to the present invention, whereby said polycation enters said cancer cells.
